(19) Europäisches Patentamt
European Patent Office
Office européen des brevets

(11) Veröffentlichungsnummer: **0 215 425**
**A2**

(12) **EUROPÄISCHE PATENTANMELDUNG**

(21) Anmeldenummer: 86112461.8

(22) Anmeldetag: 09.09.86

(51) Int. Cl.4: **C 07 D 403/04**, C 07 F 9/65, C 07 D 209/46, C 07 D 401/14, C 07 D 401/04, C 07 D 405/12, C 07 D 405/14, A 01 N 43/38, A 01 N 43/50, A 01 N 43/653, A 01 N 57/08

(30) Priorität: 19.09.85 DE 3533370
14.02.86 DE 3604599

(43) Veröffentlichungstag der Anmeldung: 25.03.87 Patentblatt 87/13

(84) Benannte Vertragsstaaten: AT BE CH DE FR GB IT LI NL SE

(71) Anmelder: HOECHST AKTIENGESELLSCHAFT, Postfach 80 03 20, D-6230 Frankfurt am Main 80 (DE)

(72) Erfinder: Liebl, Rainer, Dr., An der Weinleite 5b, D-8901 Todtenweis (DE)
Erfinder: Handte, Reinhard, Dr., Theilweg 23, D-8901 Gablingen (DE)
Erfinder: Mildenberger, Hilmar, Dr., Fasanenstrasse 24, D-6233 Kelkheim/Taunus (DE)
Erfinder: Bauer, Klaus, Dr., Kolpingstrasse 7, D-6054 Rodgau (DE)
Erfinder: Bieringer, Hermann, Dr., Eichenweg 26, D-6239 Eppstein/Taunus (DE)

(54) **Neue Tetrahydroisoindolinone, Verfahren zu ihrer Herstellung sowie ihre Verwendung im Pflanzenschutz.**

(57) Die Verbindungen der Formel I

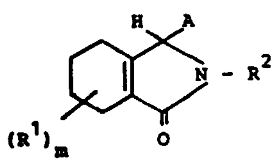

(I)

worin
$R^1$ = Alkyl, $R^2$ = Phenyl, Naphthyl, Pyridyl, Chinolinyl oder Isochinolinyl bedeutet, die substituiert sein können, einen Rest der Formeln $-C(O)-XR^3$, $-OP(Z)(YR^4)_2$ oder $-Z-(CHR^5)_2-CO-XR^3$
A = einen Rest der Formeln

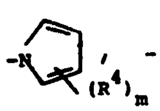, 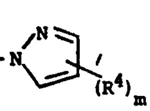, 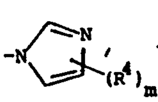, 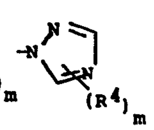,

Y
$-PR^9R^{10}$ oder -NCS und m = 0, 1 oder 2 bedeuten, besitzen vorteilhafte herbizide Eigenschaften gegenüber mono- und dikotylen Schadpflanzen.

EP 0 215 425 A2

Neue Tetrahydroisoindolinone, Verfahren zu ihrer Herstellung sowie ihre Verwendung im Pflanzenschutz

Es ist bekannt, daß bestimmte Tetrahydroisoindolinon-Derivate herbizide Eigenschaften besitzen, s. z.B. EP-A 40849, EP-A 41256. Es wurden nun neue Tetrahydroisoindoline mit vorteilhaften herbiziden Wirkungen gefunden.

Gegenstand der vorliegenden Erfindung sind daher neue 4,5,6,7-Tetrahydroisoindolinone der allgemeinen Formel I

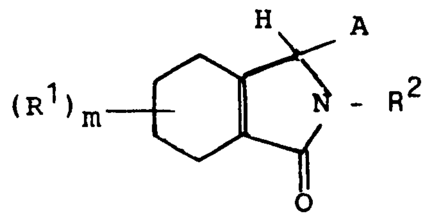

(I)

worin

$R^1$ = $(C_1-C_4)$Alkyl;

$R^2$ = Phenyl, Naphthyl, Pyridyl, Chinolinyl oder Isochinolinyl bedeutet, wobei diese Reste ein- bis vierfach, vorzugsweise ein- bis dreifach, durch gleiche oder verschiedene Reste der Gruppe Halogen, Hydroxy, $(C_1-C_4)$Alkyl, $(C_1-C_4)$Alkoxy, $(C_1-C_4)$-Alkoxy-$(C_1-C_4)$alkoxy, $(C_3-C_6)$Cycloalkoxy, $(C_3-C_6)$Alkenyloxy, $(C_3-C_6)$Alkinyloxy, Halogen-$(C_1-C_4)$alkyl, Halogen$(C_1-C_4)$alkoxy, Halogen $(C_3-C_6)$-alkenyloxy, Halogen $(C_3-C_6)$alkinyloxy, $(C_1-C_4)$-Alkylthio, $(C_1-C_4)$Alkylsulfinyl, $(C_1-C_4)$-Alkylsulfonyl, wobei bei den drei letztgenannten Resten der Alkylrest durch $(C_1-C_4)$-Alkoxycarbonyl substituiert sein kann, $NO_2$, CN, Halogen$(C_1-C_4)$alkyl, Cyano$(C_1-C_4)$alkyl, Phenoxy, Phenoxy$(C_1-C_4)$-alkyl, Phenyl$(C_1-C_4)$alkoxy, Phenylthio, Phenylsulfinyl, Phenylsulfonyl, wobei die sechs letztgenannten Reste im Phenylring bis zu dreifach durch Halogen,

$(C_1-C_4)$Alkyl, $(C_1-C_4)$Alkoxy oder $(C_1-C_4)$Alkoxycarbonyl substituiert sein können, oder durch einen Rest der Formeln

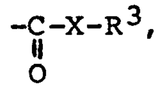, 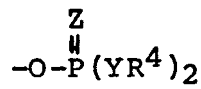 oder 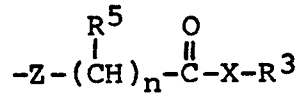

substituiert sein können,

X = O, S oder $NR^6$,

Y,Z = unabhängig voneinander O oder S ,

$R^3$ = Wasserstoff, $(C_1-C_{12})$Alkyl, das bis zu 6-fach durch Halogen und/oder bis zu zweifach durch $(C_1-C_4)$-Alkoxy, $(C_1-C_4)$Alkoxy$(C_1-C_4)$alkoxy, $(C_1-C_4$-Alkoxy)-carbonyl, $(C_1-C_4)$Alkylthio, $(C_1-C_4)$Alkylsulfinyl, $(C_1-C_4)$Alkylsulfonyl, $(C_1-C_4)$Alkylamino, Di$(C_1-C_4)$-alkylamino, Furyl, Tetrahydrofuryl, Benzofuryl, Phenyl, Phenoxy, Benzyloxy, wobei die Phenylringe bis zu dreifach durch Halogen, $(C_1-C_4)$Alkyl oder $(C_1-C_4)$Alkoxy substituiert sein können, substituiert sein kann,
$(C_3-C_6)$Cycloalkyl, $(C_3-C_6)$Alkenyl, Cyclohexenyl, $(C_3-C_6)$Alkinyl, Phenyl, das bis zu dreifach substituiert sein kann durch Halogen, $(C_1-C_4)$Alkyl, $(C_1-C_4)$Alkoxy oder $(C_1-C_4$Alkoxy)-carbonyl;
einen Rest der Formel 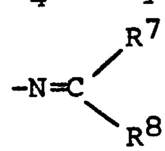

oder ein in der Landwirtschaft einsetzbares Kation;

$R^4$ = $(C_1-C_4)$Alkyl,

$R^5$ = Wasserstoff, $(C_1-C_4)$Alkyl,

$R^6$ = Wasserstoff, $(C_1-C_4)$Alkyl, das zusammen mit $R^3$ und dem diese Reste verbindenden Stickstoffatom einen fünf- bis siebengliedrigen Heterocyclus bilden kann, der als Ringglieder ein oder zwei Reste der Gruppe -O-, -S-, $NR^5$ enthält und bis zu dreifach durch $(C_1-C_4)$Alkyl substituiert sein kann,

$R^7, R^8$ =unabhängig voneinander $(C_1-C_4)$Alkyl, $(C_3-C_6)$Cycloalkyl oder gemeinsam eine $(C_2-C_7)$Alkylenkette;

A = einen Rest der Formeln

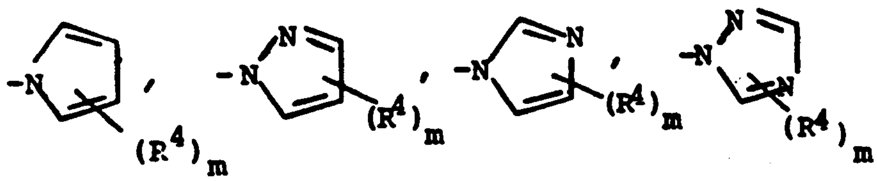

, , , ,

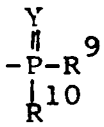 oder -NCS,

$R^9$, $R^{10}$ unabhängig voneinander $(C_1-C_4)$Alkyl, Phenyl oder Benzyl, die beide bis zu zweifach durch Halogen, $(C_1-C_4)$Alkyl, $CF_3$ oder $(C_1-C_4)$Alkoxy substituiert sein können; $(C_1-C_4)$Alkoxy; $(C_1-C_4)$-Alkylthio; OH, SH, OM oder SM, wobei M ein in der Landwirtschaft einsetzbares Kation bedeutet, oder einen Rest der Formel $-NR^{11}R^{12}$,

$R^{11}$, $R^{12}$ unabhängig voneinander Wasserstoff; $(C_1-C_6)$Alkyl; Phenyl oder Benzyl, die beide bis zu zweifach durch Halogen, $(C_1-C_4)$Alkyl, $CF_3$ oder $NO_2$ substituiert sein können, oder $R^{11}$ und $R^{12}$ zusammen mit dem benachbarten N-Atom einen fünf- bis sechsgliedrigen Heterocyclus, der als Ringglieder ein oder zwei Reste der Gruppe -O-, -S- oder $-NR^5-$ enthalten kann und der bis zu dreifach durch $(C_1-C_4)$-Alkyl substituiert sein kann;

m = 0, 1 oder 2 und

n = 1, 2 oder 3

bedeuten.

Der Rest Halogen$(C_1-C_4)$alkyl enthält insbesondere ein- bis drei Fluor-, Chlor- oder Bromatome. Halogen$(C_1-C_4)$-

alkoxy enthält insbesondere ein bis neun Fluor-, Chlor- oder Bromatome, Halogen($C_3$-$C_6$)alkenyloxy ein bis sieben Fluor-, Chlor- oder Bromatome und Halogen($C_3$-$C_6$)alkinyloxy bevorzugt ein bis fünf Fluor-, Chlor- oder Bromatome.

Die Verbindungen der Formel I können in Abhängigkeit der Reste (z.B. im Falle $R^2$ = $-Z-CH(R^5)CO-XR^3$ und $R^5 \neq H$) als reine Stereoisomeren oder deren Gemische vorliegen. Alle diese Isomerenformen werden von der Erfindung umfaßt.

Bevorzugt von den Verbindungen der Formel I sind solche Verbindungen, bei denen

$R^2$ = Phenyl, Pyridyl, die beide 1- bis 3-fach durch ($C_1$-$C_4$)Alkyl, das bis zu dreifach durch Halogen substituiert sein kann, durch F, Cl, Br, ($C_1$-$C_4$)-Alkoxy, das bis zu neunfach durch Fluor und/oder Chlor substituiert sein kann, durch ($C_1$-$C_4$)Alkylthio, $-CO-X-R^3$ oder $-Z-(\underset{R^5}{\underset{|}{C}}H)_n-CO-X-R^3$ substituiert sind,

X, Y, Z = O oder S,

$R^3$ = ($C_1$-$C_6$)Alkyl, ($C_1$-$C_4$)Alkoxycarbonyl-($C_1$-$C_4$)alkyl oder ($C_1$-$C_4$)Alkoxy-($C_1$-$C_4$)alkyl,

A = einen Rest der Formeln

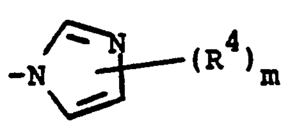 , 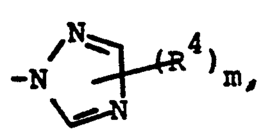 , $-\underset{R^{10}}{\underset{|}{\overset{\overset{O}{\|}}{P}}}-R^9$ oder -NCS,

$R^9$, $R^{10}$ = unabhängig voneinander ($C_1$-$C_4$)Alkyl; Phenyl oder Benzyl, die beide bis zu zweifach durch Halo-

gen, $(C_1-C_4)$Alkyl, $CF_3$ oder $(C_1-C_4)$Alkoxy substituiert sein können; $(C_1-C_4)$Alkoxy; $(C_1-C_4)$-Alkylthio; OH, SH, OM oder SM, wobei M ein in der Landwirtschaft einsetzbares Kation bedeutet, oder einen Rest der Formel $-NR^{11}R^{12}$ und

$R^{11}$, $R^{12}$ = unabhängig voneinander Wasserstoff; $(C_1-C_6)$-Alkyl; Phenyl oder Benzyl, die beide bis zu dreifach durch Halogen, $(C_1-C_4)$Alkyl, $CF_3$ oder $NO_2$ substituiert sein können oder $R^{11}$ und $R^{12}$ zusammen mit dem benachbarten N-Atom einen fünf- bis sechsgliedrigen Heterocyclus, der als Ringglieder ein oder zwei Reste der Gruppe -O-, -S- oder $-NR^5-$ enthalten kann und der bis zu dreifach durch $(C_1-C_4)$-Alkyl substituiert sein kann;

n = 1,
m = 0 oder 1 bedeuten.

Besonders bevorzugt von den Verbindungen der Formel I sind solche Verbindungen, bei denen

$R^2$ = Phenyl, das 1- bis 3-fach durch $(C_1-C_4)$Alkyl, das bis zu dreifach durch Halogen substituiert sein kann, F, Cl, Br, $(C_1-C_4)$Alkoxy, das bis zu dreifach durch Fluor und/oder Chlor substituiert sein kann, $(C_1-C_4)$Alkylthio, $-CO-X-R^3$ oder $-Z-\underset{R^5}{CH}-CO-X-R^3$ substituiert ist, wobei X, Z = O oder S, $R^3$ = $(C_1-C_6)$Alkyl, $(C_1-C_4)$-Alkoxycarbonyl-$(C_1-C_4)$alkyl oder $(C_1-C_4)$Alkoxy-$(C_1-C_4)$alkyl; $R^5$ = H oder $(C_1-C_2)$Alkyl, bedeuten,

A = einen Rest der Formel $-P(O)R^9R^{10}$, wobei

$R^9$, $R^{10}$ = unabhängig voneinander $(C_1-C_4)$Alkyl, Phenyl, OH oder OM bedeuten, und m für 0 steht.

0215425

Als Heterocyclen für den Rest $R^3$-N-$R^6$ kommen bevorzugt infrage Morpholin, Piperidin, Piperazin, Pyrrolidin.

Ein weiterer Gegenstand der Anmeldung ist ein Verfahren zur Herstellung der Verbindungen der allgemeinen Formel I, dadurch gekennzeichnet, daß man eine Verbindung der Formel II

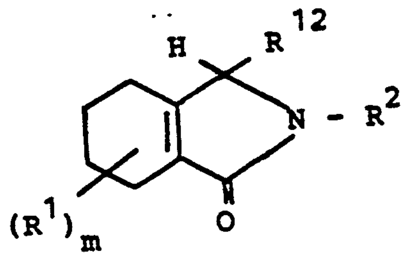

(II),

worin $R^{12}$ eine nucleofuge Gruppe wie Halogen, Tosyl oder Mesyl bedeuten, mit einer Verbindung der Formel H-A', M-SCN oder

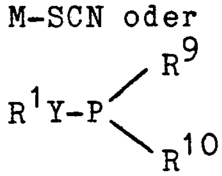

, wobei A' die Bedeutung von A außer

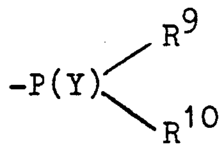

und SCN- besitzt und M ein Metallkation, bevorzugt ein Blei(II), Kalium- oder Na-Ion, bedeutet, umsetzt, und die erhaltenen Verbindungen gewünschtenfalls derivatisiert.

Die Umsetzung der Verbindung der Formel II mit der Verbindung H-A' erfolgt vorzugsweise in Gegenwart einer anorganischen Base wie z.B. Kaliumcarbonat, Natriumhydroxid oder auch Natriumhydrid oder einer organischen Base, bevorzugt einer tertiären Stickstoffbase, wie Triethylamin oder Pyridin in einem inerten organischen Lösungsmittel, wie Acetonitril oder Dimethylformamid bei Temperaturen zwischen 40 und 160°C, vorzugsweise zwischen 60 und 100°C.

Die Umsetzung von II mit einem Phosphorigsäuretriester, Phosphonigsäurediester bzw. Phosphonigsäuremonoester der Formel $R^1$-Y-$PR^9R^{10}$ erfolgt vorzugsweise ohne Lösungsmittel bei Temperaturen zwischen 50 und 140°C, gegebenenfalls unter Kaliumjodidkatalyse. Die hierbei erhaltenen Phosphon- bzw. Phosphinsäureester können gewünschtenfalls nach üblichen Methoden in die Phosphon- bzw. Phosphinsäuren überführt werden. Diese wiederum können nach geläufigen Methoden in die entsprechenden Salze, Phosphon- oder Phosphinamide überführt werden. Die Umsetzung von II mit einem Rhodanid der Formel M-SCN erfolgt in einem inerten organischen Lösungsmittel wie Toluol, Aceton oder auch Dimethylformamid bei Temperaturen zwischen 0 und 100°C, vorzugsweise bei 50 - 80°C.

Die Tetrahydroisoindolinone der Formel II sind im Falle $R^{12}$ = Cl gemäß DE-OS 28 31 770, EP-A-40849 zum Teil bekannt. Andere Verbindungen der Formel II lassen sich auf einfache Weise aus den 3-Hydroxy-4,5,6,7-tetrahydroisoindolinonen der Formel III mit einem Halogenierungsmittel, wie beispielsweise Thionylchlorid auf üblichem Wege herstellen (s.a. Deutsche Patentanmeldung P 35 33 442.8.)

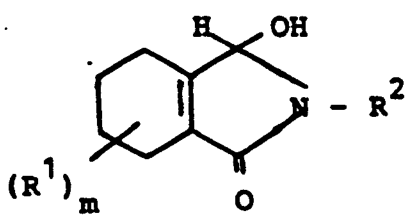

(III)

Die Verbindungen der Formel III sind ebenfalls bekannt (DE-OS 28 31 770) oder lassen sich auf einfache Weise aus den 3,4,5,6-Tetrahydrophthalimiden der Formel IV mit einem Reduktionsmittel wie $NaBH_4$, in bekannter Weise herstellen, (s. Rocz. Chem. 49, 1671 (1975).

 

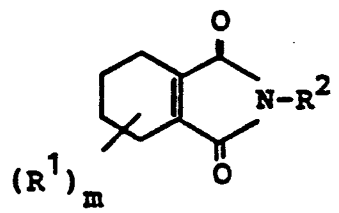

(IV)

Die Verbindungen der Formel IV wiederum lassen sich aus 3,4,5,6-Tetrahydrophthalsäureanhydriden und den entsprechenden Aminen nach üblichen Methoden herstellen.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden.

Beispielsweise können folgende Schadpflanzen bekämpft werden:
Schadgräser wie
Avena fatua,
Alopecurus sp.
Lolium sp.
Setaria sp.
Digitaria sp.
Sorghum halepense
Echinochloa sp.
Agropyron sp.
Cynodon sp.
Phalaris sp.,

<u>dikotyle Pflanzen</u> wie
Lamium sp.
Veronica sp.

Galium sp.
Stellaria sp.
Matricaria sp.
Papaver sp.
Centauria sp.
Amaranthus sp.
Galinsoga sp.
Mercurialis sp.
Sida sp.
Abutilon sp.
Ambrosia sp.
Xanthium sp.
Cirsium sp.
Artemisia sp.
Rumex sp.
Convolvulus sp.
Ipomea sp.
Sinapis sp.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis fünf Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wachstumsstadium stehen oder sterben nach einer gewissen Zeit mehr oder weniger schnell ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichente herbizide Aktivität gegenüber mono- und dikotylen Unkräutern aufweisen, werden Kulturpflanzen wirtschaftlich bedeutender Kulturen wie z. B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z. B. durch Auslösen von Desikkation, Abszission und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Mittel können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z. B. polyoxethylierte Alkylphenole, polyoxethylierte Fettalkohole, Alkyl- oder Alkylphenylsulfonate und Dispergiermittel, z. B. ligninsulfonsaures Natrium, 2,2'-di-

0215425

naphthylmethan-6,6'-disulfonsaures Natrium, dibutylnaphthalinsulfonsaures Natrium oder auch oleoylmethyltaurinsaures Natrium enthalten. Die Herstellung erfolgt in üblicher Weise, z. B. durch Mahlen und Vermischen der Komponenten.

Emulgierbare Konzentrate können z. B. durch Auflösen des Wirkstoffes in einem inerten organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z. B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z. B. Polyvinylalkohol, poyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvernbeträgt die Wirkstoffkonzentration z. B. etwa 10 bis 90 Gew.-%, der Rest zu 100 Gew.-% besteht aus üblichen Formulierungsbestandteilen., Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u. a. variiert die erforderliche Aufwandmenge. Sie kann innerhalb weiter Grenzen schwanken, z. B. zwischen 0,005 und 10,0 gk/ha oder mehr Aktivsubstanz, vorzugsweise liegt sie jedoch zwischen 0,01 und 2 kg/ha.

Auch Mischungen oder Mischformulierungen mit anderen Wirkstoffen, wie z. B. Insektiziden, Akariziden, Herbiziden, Düngemitteln, Wachstumsregulatoren oder Fungiziden sind gegebenenfalls möglich.

Die Erfindung wird durch nachstehende Beispiele erläutert.

A. Formulierungsbeispiele

Eine Stäubemittel wird erhalten, indem man 10 Gewichtsteile Wirkstoff und 90 Gewichtsteile Talkum oder Inertstoff mischt und in einer Schlagmühle zerkleinert.

Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat wird erhalten, indem man 20 Gewichtsteile Wirkstoff mit 6 Gewichtsteilen Alkylphenolpolyglykolether (®Triton X 207), 3 Gewichtsteilen Isotridecanolpolyglykolether (8 AeO) und 71 Gewichtsteilen paraffinischem Mineralöl (Siedebereich z. B. ca. 255 bis über 377 °C) mischt und in einer Reibkugelmühle auf eine Feinheit von unter 5 Mikron vermahlt.

Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

## B. Chemische Beispiele

### Beispiel 1

**2-(4-Chlor-2-fluor-5-methoxy-phenyl)-3-imidazol-1-yl-4,5,6,7-tetrahydroisoindolinon**

1,36 g (20,0 mmol) Imidazol und 2,76 g (20,0 mmol) Kaliumcarbonat werden in 40 ml Acetonitril auf 60 °C erwärmt. Bei 60 °C tropft man 6,40 g (20,0 mmol) 3-Chlor-2-(4-chlor-2-fluor-5-methoxy-phenyl)-4,5,6,7-tetrahydroisoindolinon, in 20 ml Acetonitril gelöst, zu. Man rührt 4 h bei 60 - 70 °C bis die Umsetzung beendet ist. Der Ansatz wird auf 250 ml Wasser gegossen und mit 100 ml Methylenchlorid extrahiert. Die Methylenchloridphase wird nochmals mit 100 ml Wasser gewaschen und über Natriumsulfat getrocknet. Nach dem Abdestillieren des Lösungsmittels wird der verbleibende feste Rückstand mit Petrolether verrieben und abgesaugt. Man erhält 5,6 g (80,0 % d. Th.) 2-(4-Chlor-2-fluor-5-methoxy-phenyl)-3-imidazol-1-yl-4,5,6,7-tetrahydroisoindolinon in Form von hellbraunen Kristallen mit Schmp. 78 - 85 °C.

### Beispiel 2

**2-(4-Chlorphenyl)-3-(1,2,4-triazol-1-yl)-4,5,6,7-tetrahydroisoindolinon**

1,38 g (20,0 mmol) Triazol und 2,76 g (20,0 mmol) Kaliumcarbonat werden in 40 ml Acetonitril auf 60 °C erwärmt. Bei 60 °C tropft man 5,64 g (20,0 mmol) 3-Chlor-2-(4-chlorphenyl)-4,5,6,7-tetrahydroisoindolinon, in 20 ml Acetonitril gelöst, zu. Man rührt bei 70 °C bis die Umsetzung beendet ist (5 h) und gießt auf 250 ml Wasser. Man extrahiert mit 100 ml Methylenchlorid, wäscht die Methylenchloridphase mit 100 ml Wasser und trocknet über Natriumsulfat. Nach dem Abdestillieren des Lösungsmittels wird der verbleibende feste Rückstand

mit Petrolether verrieben und abgesaugt. Man erhält 5,40 g (86 % d. Th.) 2-(4-Chlorphenyl)-3-(1,2,4-triazol-1-yl)-4,5,6,7-tetrahydroisoindolinon in Form hellgelber Kristalle mit Schmp. 64 - 68 °C.

## Beispiel 3

2-(4-Chlor-2-fluor-5-isopropoxy-phenyl)-3-diethoxyphosphoryl-4,5,6,7-tetrahydroisoindolinon

17,9 g (0,05 mol) 3-Chlor-2-(4-chlor-2-fluor-5-isopropoxy-phenyl)-4,5,6,7-tetrahydroisoindolinon und 10,0 g (0,06 mol) Triethylphosphit werden mit 30 mg Kaliumjodid auf 70 °C erwärmt. Nach Beendigung der Gasentwicklung wird noch 30 min auf 100 °C erhitzt. Das beim Abkühklen teilweise kristallierende Reaktionsgemisch wird mit 150 ml Ether erhitzt und der Niederschlag abgesaugt. Man erhält 21,2 g (92 % d. Th.) 2-(4-Chlor-2-fluor-5-isopropoxy-phenyl)-3-diethylphosphoryl-4,5,6,7-tetrahydroisoindolinon in Form farbloser Kristalle mit Schmp. 108 - 110 °C.

## Beispiel 4

2-(4-Chlor-2-fluor-5-methoxy-phenyl)-3-rhodano-4,5,6,7-tetrahydroisoindolinon

6,4 g (0,02 mol) 3-Chlor-2-(4-chlor-2-fluor-5-methoxy-phenyl)-4,5,6,7-tetrahydroisoindolinon und 3,2 g (0,01 mol) Blei(II)rhodanid werden in 80 ml Toluol 3 h auf 70 °C erwärmt. Man saugt vom gebildeten Bleichlorid ab, wäscht die Toluolphase kurz mit 100 ml Wasser, trocknet über Natriumsulfat und destilliert das Lösungsmittel ab. Der Rückstand wird bei 40 °C am Vakuum bis zur Gewichtskonstanz getrocknet. Man erhält 6,3 g (92 % d. Th.) 2-(4-Chlor-2-fluor-5-methoxy-phenyl)-3-rhodano-4,5,6,7-tetrahydroisoindolinon in Form eines zähen gelben Öls.

## Beispiel 5

2-(2-Chlor-4-fluor-5-methoxy-phenyl)-3-pyrrolyl-4,5,6,7-tetrahydroisoindolinon

Man löst 1,34 g (0,02 mol) Pyrrol und 2,0 g (0,02 mol) Triethylamin in 40 ml Toluol und tropft bei RT 6,4 g (0,02 mol) 3-Chlor-2-(4-chlor-2-fluor-5- methoxy-phenyl)-4,5,6,7-tetrahydroisoindolinon, gelöst in 30 ml Toluol, zu. Man rührt 3 h bei 70 °C, wäscht zweimal mit je 100 ml Wasser und trocknet über Natriumsulfat. Nach dem Abziehen des Lösungsmittels erhält man 6,20 g (89 % d. Th.) 2-(2-Chlor-4-fluor-5-methoxy-phenyl)-3-pyrrolo-4,5,6,7-tetrahydroisoindolinon in Form eines braunen Öls.

In analoger Weise lassen sich die Verbindungen der folgenden Tabelle 1 herstellen.

0215425

Tabelle 1

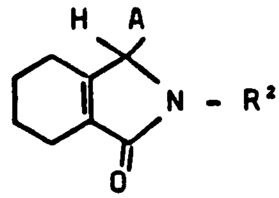

| Bsp. | A | $R^2$ | Fp [° C] | Herstellung analog Bsp. |
|---|---|---|---|---|
| 6 | I**) | $4-Cl-C_6H_4$ | 68-72 | 1 |
| 7 | T*) | $3,5-Cl_2-C_6H_3$ | 76-80 | 2 |
| 8 | T | $4-Cl-2-F-5-OCH(CH_3)_2-C_6H_2$ | hellbraunes Harz | 2 |
| 9 | T | $2,4-Cl_2-C_6H_3$ | 68-75 | 2 |
| 10 | I | $2,4-Cl_2-C_6H_3$ | 72-76 | 1 |
| 11 | I | $2,4-Cl_2-5-OCH_3-C_6H_2$ | | 1 |
| 12 | T | $2,6-(C_2H_5)_2-C_6H_3$ | gelbes Oel | 2 |
| 13 | T | $3-(C_6H_5O)-C_6H_4$ | | 2 |
| 14 | T | $4-(4-Cl-C_6H_4O)-C_6H_4$ | gelbes Harz | 2 |
| 15 | T | $4-(4-Cl-C_6H_4-O-CH_2)-C_6H_4$ | | 2 |
| 16 | T | $2,4-F_2-5-CO_2C_2H_5-C_6H_2$ | | 2 |
| 17 | I | $2,4-Cl_2-5-CO_2C_2H_5-C_6H_2$ | | 1 |
| 18 | I | $2,4-Cl_2-5-OCH_3-C_6H_2$ | | 1 |
| 19 | T | $2,4-F_2-C_6H_3$ | | 2 |
| 20 | I | " | hellbraunes Öl | 1 |
| 21 | T | $2,4-Cl_2-5-CO_2CH_2CO_2CH_3-C_6H_2$ | | 2 |
| 22 | I | $2,4-F_2-3,5-Cl_2-C_6H$ | | 1 |
| 23 | T | " | | 2 |
| 24 | T | $(3,5-CF_3)_2-C_6H_3$ | | 2 |
| 25 | T | $(3,5-CO_2CH_3)_2-C_6H_3$ | | 2 |
| 26 | T | $2,4-Cl_2-5-CN-C_6H_2$ | | 2 |
| 27 | T | 1-naphthyl | | 2 |

| Bsp. | A | $R^2$ | Fp [° C] | Herstellung analog Bsp. |
|---|---|---|---|---|
| 28 | I | 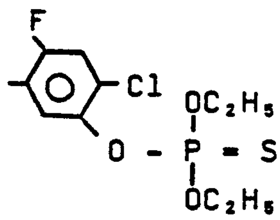 | | 1 |
| 29 | T | " | | 2 |
| 30 | T | $2,4-Cl_2-5-SO_2CH_3-C_6H_2$ | | 2 |
| 31 | T | 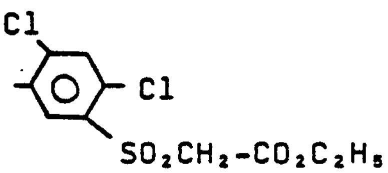 | | 2 |
| 32 | T | $3-CO_2C_2H_5-4-Cl-C_6H_3$ | | 2 |
| 33 | I | $4-Cl-3-OCH(CH_3)_2-C_6H_3$ | | 1 |
| 34 | I | 4-Cl-pyrid-3-yl | | 1 |
| 35 | T | " | | 2 |
| 36 | 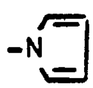 | $4-Cl-2-F-5-OCH_3-C_6H_2$ | | 5 |
| 37 | " | $2,4-Cl_2-5-OCH_3-C_6H_2$ | | 5 |
| 38 | " | $2,4-Cl_2-C_6H_3$ | | 5 |
| 39 | " | $4-Cl-C_6H_4$ | | 5 |
| 40 | " | $2,4-F_2-C_6H_3$ | | 5 |
| 41 | " | $2,4-F_2-5-CO_2C_2H_5-C_6H_2$ | | 5 |
| 42 | " | $2,4-Cl_2-5-CO_2C_2H_5-C_6H_2$ | | 5 |
| 43 | " | $4-Cl-3-CO_2C_2H_5-C_6H_3$ | | 5 |
| 44 | " | $4-Cl-3-OCH(CH_3)_2-C_6H_3$ | | 5 |
| 45 | " | $4-F-C_6H_4$ | | 5 |
| 46 | " | 4-Cl-pyrid-3-yl | | 5 |
| 47 | " | 1-naphthyl | | 5 |
| 48 | " | 8-chinolinyl | | 5 |

| Bsp. | A | $R^2$ | Fp[°C] | Herstellg analog Bsp. |
|---|---|---|---|---|
| 49 | $\overset{O}{\overset{\|}{P}}(OC_2H_5)_2$ | $4\text{-}Cl\text{-}2\text{-}F\text{-}5\text{-}OCH_3\text{-}C_6H_2$ | 128-31 | 3 |
| 50 | " | $4\text{-}Cl\text{-}C_6H_4$ | 112-13 | 3 |
| 51 | " | $3,5\text{-}Cl_2\text{-}C_6H_3$ | 181-84 | 3 |
| 52 | " | $3,5\text{-}(CF_3)_2\text{-}C_6H_3$ | 175-76 | 3 |
| 53 | " | $2,4\text{-}F_2\text{-}C_6H_3$ | gelbes Oel | 3 |
| 54 | $\overset{O}{\overset{\|}{P}}(OCH_3)_2$ | " | " | 3 |
| 55 | " | $4\text{-}F\text{-}C_6H_4$ | 106-10 | 3 |
| 56 | $\overset{O}{\overset{\|}{P}}(OC_2H_5)_2$ | $4\text{-}Cl\text{-}C_6H_4O\text{-}C_6H_4$ | 125-26 | 3 |
| 57 | $\overset{O}{\overset{\|}{P}}(OCH_3)_2$ | $3\text{-}C_6H_5O\text{-}C_6H_4$ | 102-04 | 3 |
| 58 | " | $4\text{-}(4\text{-}Cl\text{-}C_6H_4\text{-}CH_2O)\text{-}C_6H_4$ | | 3 |
| 59 | " | $4\text{-}Cl\text{-}2\text{-}F\text{-}5\text{-}OCH_3\text{-}C_6H_2$ | | 3 |
| 60 | " | $4\text{-}Cl\text{-}2\text{-}F\text{-}5\text{-}OCH(CH_3)_2\text{-}C_6H_2$ | | 3 |
| 61 | " | $2,4\text{-}F_2\text{-}5\text{-}CO_2C_2H_5\text{-}C_6H_2$ | | 3 |
| 61 | $\overset{O}{\overset{\|}{P}}(OC_2H_5)_2$ | " | | 3 |
| 63 | " | $2,4\text{-}Cl_2\text{-}5\text{-}CO_2C_2H_5\text{-}C_6H_2$ | gelbes Öl | 3 |
| 64 | " | $2,4\text{-}Cl_2\text{-}5\text{-}CO_2C_4H_9\text{-}C_6H_2$ | | 3 |
| 65 | " | $2,4\text{-}Cl_2\text{-}5\text{-}CO_2CH_2CO_2CH_3\text{-}C_6H_2$ | | 3 |
| 66 | $\overset{O}{\overset{\|}{P}}(OCH_3)_2$ | $2,4\text{-}Cl_2\text{-}5\text{-}CO_2CH(CH_3)CO_2C_2H_5\text{-}C_6H_2$ | | 3 |
| 67 | " | $4\text{-}Cl\text{-}3\text{-}CO_2C_2H_5\text{-}C_6H_3$ | 119-21 | 3 |
| 68 | " | $4\text{-}Cl\text{-}3\text{-}OCH(CH_3)_2\text{-}C_6H_3$ | | 3 |
| 69 | " | 4-Cl-pyrid-3-yl | | 3 |
| 70 | " | 3-pyridyl | | 3 |
| 71 | $\overset{O}{\overset{\|}{P}}(OC_2H_5)_2$ | 4-Cl-pyrid-3-yl | | 3 |
| 72 | " | $3,5\text{-}(CO_2CH_3)_2\text{-}C_6H_3$ | | 3 |
| 73 | " | $2,4\text{-}Cl_2\text{-}5\text{-}CN\text{-}C_6H_2$ | | 3 |
| 74 | " | $2,4\text{-}Cl_2\text{-}5\text{-}SO_2CH_3\text{-}C_6H_2$ | | 3 |

| Bsp. | A | $R^2$ | Fp[°C] | Herstellg. analog Bsp. |
|---|---|---|---|---|
| 75 | $\overset{\underset{\|}{O}}{P}(OCH_3)_2$ | 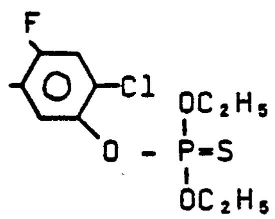 | | 3 |
| 76 | " | $2,4Cl_2-5-SO_2CH_2CO_2C_2H_5-C_6H_2$ | | 3 |
| 77 | " | $2,6-(C_2H_5)_2-C_6H_3$ | gelbes Oel | 3 |
| 78 | " | 1-naphthyl | | 3 |
| 79 | " | 8-chinolinyl | | 3 |
| 80 | " | 5-isochinolinyl | | 3 |
| 81 | " | 1-isochinolinyl | | 3 |
| 82 | -SCN | $4-Cl-2-F-5-OCH(CH_3)_2-C_6H_2$ | gelbes Oel | 4 |
| 83 | " | $2,4-Cl_2-5-OCH_3-C_6H_2$ | " | 4 |
| 84 | " | $2,4-Cl_2-C_6H_3$ | | 4 |
| 85 | " | $4-Cl-C_6H_4$ | 105-07 | 4 |
| 86 | " | $4-F-C_6H_4$ | | 4 |
| 87 | " | $2,4-F_2-C_6H_3$ | oranges Öl | 4 |
| 88 | " | $4-Cl-3-CO_2C_2H_5-C_6H_3$ | | 4 |
| 89 | " | $3-CO_2C_2H_5-C_6H_4$ | | 4 |
| 90 | " | $4-Cl-3-OCH(CH_3)_2-C_6H_3$ | | 4 |
| 91 | " | $2,4-Cl_2-5-CO_2C_2H_5-C_6H_2$ | | 4 |
| 92 | " | $2,4-F_2-5-CO_2C_2H_5-C_6H_2$ | | 4 |
| 93 | " | 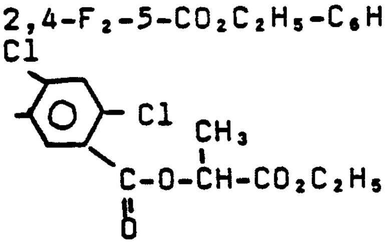 | | 4 |
| 94 | " | 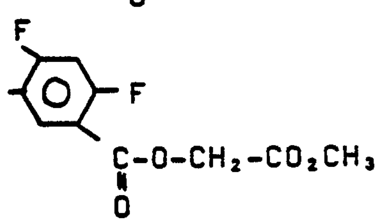 | | 4 |

| Bsp. | A | $R^2$ | Fp[°C] | Herstellg. analog Bsp. |
|---|---|---|---|---|
| 95 | SCN | $3,5-Cl_2-C_6H_3$ | | 4 |
| 96 | " | $3,5-(CO_2CH_3)_2-C_6H_3$ | | 4 |
| 97 | " | $3,5-(CF_3)_2-C_6H_3$ | | 4 |
| 98 | " | $3,5-Cl_2-2,4-F_2-C_6H$ | | 4 |
| 99 | " | $4-C_6H_5-O-C_6H_4$ | | 4 |
| 100 | " | $3-C_6H_5O-C_6H_4$ | | 4 |
| 101 | " | $4-(4-Cl-C_6H_4-CH_2O)-C_6H_4$ | | 4 |
| 102 | " | 1-naphthyl | | 4 |
| 103 | " | 3-pyridyl | | 4 |
| 104 | " | 8-chinolinyl | | 4 |
| 105 | " | 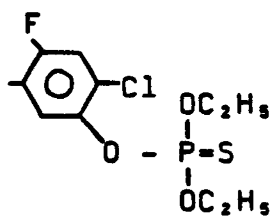 | | 4 |
| 106 | " | $2,4-Cl_2-5-SO_2CH_3-C_6H_2$ | | 4 |
| 107 | " | $2,4-Cl_2-5-CN-C_6H_2$ | | 4 |
| 108 | $P(O)-(OC_2H_5)_2$ | $2,4-Cl_2-5-OCH_3-C_6H_2$ | 164-66 | 3 |
| 109 | T | $4-Cl-2-F-5-OCH_3-C_6H_2$ | hellbraunes Harz | 2 |
| 110 | I | $3-CF_3-C_6H_4$ | hellbraunes Harz | 1 |
| 111 | I | $4-Cl-3-OCH_3-C_6H_3$ | " | 1 |
| 112 | I | $4-Cl-3-CO_2C_2H_5-C_6H_3$ | " | 1 |
| 113 | T | $4-Cl-3-OCH_2CO_2CH_3-C_6H_3$ | " | 2 |
| 114 | $P(O)(OCH_3)_2$ | $2,4-Cl_2-5-CO_2C_2H_5-C_6H_2$ | 119-122 | 3 |
| 115 | $P(O)(OC_2H_5)_2$ | $2-Cl-5-CO_2C_2H_5-C_6H_3$ | gelbes Öl | 3 |
| 116 | $P(O)(OC_2H_5)_2$ | $3-CO_2CH(CH_3)_2-C_6H_4$ | hellbraunes Harz | 3 |

| Bsp. | A | $R^2$ | Fp [°C] | Herstellg. analog Bsp. |
|---|---|---|---|---|
| 117 | $\overset{O}{P}(OC_2H_5)_2$ | $3-CF_3-C_6H_4$ | 98-101 | 3 |
| 118 | SCN | $3-CF_3-C_6H_4$ | hellbraunes Harz | 4 |
| 119 | $\overset{O}{P}(OC_2H_5)_2$ | $4-Br-3-CO_2C_2H_5-C_6H_3$ | | 3 |
| 120 | $\overset{O}{P}(OC_2H_5)_2$ | $4-Br-3-CO_2CH(CH_3)_2-C_6H_3$ | | 3 |
| 121 | SCN | $4-Br-3-CO_2C_2H_5-C_6H_3$ | gelbes Öl | 4 |
| 122 | $\overset{O}{P}(OC_2H_5)_2$ | $4-CF_3-C_6H_4$ | 148-150 | 3 |
| 123 | $\overset{O}{P}(OC_2H_5)_2$ | $4-Cl-3-OCH_3-C_6H_3$ | 120-121 | 3 |
| 124 | $\overset{O}{P}(OC_2H_5)_2$ | $4-Cl-2-F-5-OCH_2CO_2CH_3-C_6H_2$ | | 3 |
| 125 | $\overset{O}{P}(OC_2H_5)_2$ | $4-Cl-2F-5-O-\overset{CH_3}{CH}-CO_2C_2H_5-C_6H_2$ | | 3 |
| 126 | $\overset{O}{P}(OCH_3)_2$ | $4-Cl-2F-5-O-\overset{CH_3}{CH}-CO_2C_2H_5-C_6H_2$ | | 3 |
| 127 | SCN | $4-Cl-2F-5-O-\overset{CH_3}{CH}-CO_2C_2H_5-C_6H_2$ | gelbes Öl | 4 |
| 128 | SCN | $4-Cl-2F-5-O-CH_2-CO_2CH_3-C_6H_2$ | gelbes Öl | 4 |

*) T = 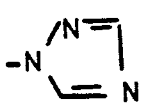

**) I = 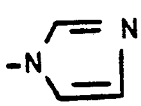

Beispiel 129

Monoethyl-[2-(4-chlor-2-fluor-5-methoxy-phenyl)-3-oxo-2,3,4,5,6,7-hexahydro-1H-isoindol-1-yl]-phosphonat

8,6 g (20 mmol) 2-(4-Chlor-2-fluor-5-methoxy-phenyl)-3-diethoxy-phosphoryl-4,5,6,7-tetrahydroisoindolinon (Bsp. 49) werden 4 h bei 100 °C in 80 ml 10%iger Salzsäure kräftig gerührt. Die wäßrige Salzsäure wird bei 50 °C am Wasserstrahlvakuum abdestilliert. Der harzige Rückstand wird in 100 ml 5%iger Kaliumhydroxidlösung gelöst und mit 50 ml Essigester geschüttelt. Die Wasserphase wird unter Eiskühlung mit konzentrierter Salzsäure auf pH 2 gebracht und der ausgefallene Niederschlag abgesaugt und getrocknet. Man erhält 6,2 g (77 % d. Th.) Monoethyl[2-(4-chlor-2-fluor-5-methoxy-phenyl)-3-oxo-2,3,4,5,6,7-hexahydro-1H-isoindol-1-yl]-phosphonat in Form farbloser Kristalle mit Schmp. 124 - 126 °C.

Beispiel 130

Monoethyl[2-(4-Chlor-2-fluor-5-methoxy-phenyl)-3-oxo-2,3,4,5,6,7-hexahydro-1H-isoindol-1-yl]-phosphonat-Kaliumsalz

8,5 g (20 mmol) Monoethyl[2-(4-chlor-2-fluor-5-methoxy-phenyl)-3-oxo-2,3,4,5,6,7-hexahydro-1H-isoindol-1-yl]-phosphonat (Bsp. 129) und 1,12 g (20 mmol) Kaliumhydroxid werden 30 min in 100 ml Methanol bei 20 °C gerührt. Man zieht das Lösungsmittel ab und trocknet den Rückstand. Man erhält 9,4 g (100 % d. Th.) Monoethyl[2-(4-chlor-2-fluor-5-methoxy-phenyl)-3-oxo-2,3,4,5,6,7-hexahydro-1H-isoindol-1-yl]-phosphonat-Kaliumsalz in Form hellgelber Kristalle, die bei 151 - 153 °C unter Zersetzung schmelzen.

Beispiel 131

[2-(4-Chlorphenyl)-3-oxo-2,3,4,5,6,7-hexahydro-1H-isoindol-1-yl]-phosphonsäure

19,2 g (0,05 mol) 2-(4-Chlorphenyl)-3-diethoxyphosphoryl-4,5,6,7-tetrahydroisoindolinon (Bsp. 50) wird 2 h in 130 ml konz. Bromwasserstoffsäure bei 100 °C gerührt, einrotiert und bei 50 °C am Hochvakuum getrocknet. Man erhält 16,1 g (98 % d. Th.) [2-(4-Chlorphenyl)-3-oxo-2,3,4,5,6,7-hexahydro-1H-isoindol-1-yl]-phosphonsäure in Form farbloser Kristalle mit Schmp. 103 - 106 °C.

Beispiel 132

[2-(4-Chlorphenyl)-3-oxo-2,3,4,5,6,7-hexahydro-1H-isoindol-1-yl]-phosphonsäure-dikaliumsalz

Man löst 16,4 g (0,05 mol) [2-(4-Chlorphenyl)-3-oxo-2,3,4,5,6,7-hexahydro-1H-isoindol-1-yl]-phosphonsäure (Bsp. 131) und 5,6 g (0,10 mol) Kaliumhydroxid in 200 ml Methanol und rührt 30 min bei 20 °C. Man destilliert das Methanol ab und trocknet den Rückstand. Man erhält 22,0 g (100 % d. Th.) [2-(4-Chlorphenyl)-3-oxo-2,3,4,5,6,7-hexahydro-1H-isoindol-1-yl]-phosphonsäure-dikaliumsalz in Form gelber Kristalle, die bei 188 - 190 °C unter Zersetzung schmelzen.

Beispiel 133

Ethyl[2-(4-chlor-2-fluor-5-methoxy-phenyl)-3-oxo-2,3,4,5,6,7-hexahydro-1H-isoindol-1-yl] methyl -phosphinat

9,9 g (0,03 mol) 3-Chlor-2-(4-chlor-2-fluor-5-methoxy-phenyl)-4,5,6,7-tetrahydroisoindolinon und 4,1 g (0,03 mol) Methanphosphorigsäurediethylester werden zusammen mit 10 mg Kaliumjodid 20 min auf 120 °C erhitzt. Der erhaltene Feststoff wird mit 100 ml Ether aufgekocht und abgesaugt.Man erhält 11,4 g (95 % d. Th.) Ethyl[2-(4-chlor-2-fluor-5-methoxy-phenyl)-3-oxo-2,3,4,5,6,7-hexahydro-1H-isoindol-1-yl] methyl -phosphinat in Form farbloser Kristalle mit Schmp. 180-183 °C.

Beispiel 134

[2-(4-Chlor-2-fluor-5-methoxy-phenyl)-3-oxo-2,3,4,5,6,7-hexahydro-1H-isoindol-1-yl],methyl- phosphinsäure

12,1 g (0,03 mol) Ethyl[2-(4-chlor-2-fluor-5-methoxy-phenyl)-3-oxo-2,3,4,5,6,7-hexahydro-1H-isoindol-1-yl,]methyl -phosphinat (Bsp. 133) wird 3 h bei 100 °C in 120 ml 20%iger Salzsäure gerührt und einrotiert. Das verbleibende Harz wird in 100 ml 10%iger Kaliumhydroxidlösung gelöst und mit 50 ml Essigester geschüttelt. Die Wasserphase wird unter Eiskühlung mit konz. Salzsäure auf pH 2 gebracht und der ausgefallene Niederschlag abgesaugt und getrocknet. Man erhält 10,4 g (93 % d. Th.) [2-(4-Chlor-2-fluor-5-methoxy-phenyl)-3-oxo-2,3,4,5,6,7-hexahydro-1H-isoindol-1-yl],methyl- phosphinsäure in Form farbloser Kristalle mit Schmp. 153 - 155 °C.

Beispiel 135

[2-(4-Chlor-2-fluor-5-methoxy-phenyl)-3-oxo-2,3,4,5,6,7-hexahydro-1H-isoindol-1-yl], methyl-phosphinsäure-Kaliumsalz

7,5 g (0,02 mol) [2-(4-Chlor-2-fluor-5-methoxy-phenyl)-3-oxo-2,3,4,5,6,7-hexahydro-1H-isoindol-1-yl],methyl-phosphinsäure (Bsp. 134) und 1,12 g (0,02 mol) Kaliumhydroxid werden in 100 ml Methanol gelöst und 20 min bei 20 °C gerührt. Man zieht das Lösungsmittel ab und trocknet den Rückstand bei 50 °C im Hochvakuum. Man erhält 8,3 g (100 % d. Th.) [2-(4-Chlor-2-fluor-5-methoxy-phenyl)-3-oxo-2,3,4,5,6,7-hexahydro-1H-isoindol-1-yl],methyl-phosphinsäure-Kaliumsalz in Form hellgelber Kristalle die bei 174 - 177 °C unter Zersetzung schmelzen.

## Beispiel 136

**2-(4-Chlor-2-fluor-5-methoxy-phenyl)-3-diphenylphosphoryl-4,5,6,7-tetrahydroisoindolinon**

9,9 g (0,03 mol) 3-Chlor-2-(4-chlor-2-fluor-5-methoxy-phenyl)-4,5,6,7-tetrahydroisoindolinon und 6,9 g (0,03 mol) Diphenylphosphinigsäureethylester wird mit 20 mg Kaliumjodid 30 min auf 120 °C erwärmt. Der verbleibende Rückstand wird mit 150 ml Ether aufgekocht und abgesaugt. Man erhält 13,4 g (90 % d. Th.) 2-(4-Chlor-2-fluor-5-methoxy-phenyl)-3-diphenylphosphoryl-4,5,6,7-tetrahydroisoindolinon in Form farbloser Kristalle mit Schmp. 207 - 209 °C.

**Fortsetzung Tabelle 1**

| Bsp. | A | $R^2$ | Fp [°C] | Herstellung analog Bsp. |
|---|---|---|---|---|
| 137 | $-\overset{O}{\overset{\|}{P}}(OH)_2$ | $4-Cl-2-F-5-OCH_3-C_6H_2$ | | 131 |
| 138 | $-\overset{O}{\overset{\|}{P}}(OK)_2$ | " | | 132 |
| 139 | $-\overset{O}{\overset{\|}{P}}(OC_2H_5)_2$ | $4-Br-C_6-H_4$ | 128-131 | 3 |
| 140 | $-\overset{O}{\overset{\|}{P}}(OC_2H_5)_2$ | $4-J-C_6H_4$ | 150-153 | 3 |
| 141 | $-\underset{OH}{\overset{O}{\overset{\|}{P}}}-OC_2H_5$ | " | 126-128 | 129 |
| 142 | $-\underset{OC_2H_5}{\overset{O}{\overset{\|}{P}}}-OK$ | $4-Cl-C_6H_4$ | 151-153 | 130 |
| 143 | $-\overset{O}{\overset{\|}{P}}(OH)_2$ | $4-Br-C_6H_4$ | 143-145 | 131 |
| 144 | $-\underset{CH_3}{\overset{O}{\overset{\|}{P}}}-OC_2H_5$ | $4-Cl-C_6H_4$ | | 133 |
| 145 | " | $4-Br-C_6H_4$ | | 133 |

| Bsp. | A | $R^2$ | Fp [°C] | Herstellung anlago Bsp. |
|---|---|---|---|---|
| 146 | O<br>"<br>$-P-OC_2H_5$<br>$CH_3$ | $3-CO_2C_2H_5-4-Cl-C_6H_3$ | | 133 |
| 147 | O<br>"<br>$-P-OH$<br>$CH_3$ | $4-Cl-C_6H_4$ | | 134 |
| 148 | " | $4-Br-C_6H_4$ | | 134 |
| 149 | O<br>"<br>$-P-OK$<br>$CH_3$ | $4-Cl-C_6H_4$ | | 135 |
| 150 | " | $4-Br-C_6H_4$ | | 135 |
| 151 | O<br>"<br>$-P(CH_3)_2$ | $4-Cl-2-F-5-OCH_3-C_6H_2$ | | 136 |
| 152 | O<br>"<br>$-P(CH_3)_2$ | $4-Cl-C_6H_4$ | | 136 |
| 153 | " | $4-Br-C_6H_4$ | | 136 |
| 154 | O<br>"<br>$-P-C_6H_5$<br>$CH_3$ | $4-Cl-C_6H_4$ | | 136 |
| 155 | O<br>"<br>$-P-C_6H_5$<br>$CH_3$ | $4-Cl-2-F-5-OCH_3-C_6H_2$ | | 136 |
| 156 | O<br>"<br>$-P-OCH_3$<br>OH | " | | 129 |
| 157 | " | $4-Cl-C_6H_4$ | | 129 |
| 158 | " | $4-Br-C_6H_4$ | | 129 |
| 159 | O<br>"<br>$-P-OK$<br>$OCH_3$ | $4-Cl-2-F-5-OCH_3-C_6H_2$ | | 130 |
| 160 | " | $4-Cl-C_6H_4$ | | 130 |
| 161 | " | $4-Br-C_6H_4$ | | 130 |

| Bsp. | A | $R^2$ | Fp[°C] | hergestellt analog Bsp. |
|---|---|---|---|---|
| 162 | $\overset{\overset{O}{\parallel}}{P}(OCH_3)_2$ | $4\text{-}Cl\text{-}3\text{-}CO_2C_4H_9\text{-}C_6H_3$ | Harz | 3 |
| 163 | " | $4\text{-}F\text{-}3\text{-}CO_2C_2H_5\text{-}C_6H_3$ | | 3 |
| 164 | " | $4\text{-}Br\text{-}3\text{-}CO_2C_2H_5\text{-}C_6H_3$ | | 3 |
| 165 | $\overset{\overset{O}{\parallel}}{P}(OC_2H_5)_2$ | $2,4\text{-}F_2\text{-}5\text{-}CO_2C_2H_5\text{-}C_6H_2$ | | 3 |
| 166 | " | $4\text{-}Cl\text{-}3\text{-}CO_2C_2H_5\text{-}C_6H_3$ | | 3 |
| 167 | " | $4\text{-}Br\text{-}3\text{-}CO_2C_3H_7\text{-}C_6H_3$ | | 3 |
| 168 | " | $4\text{-}F\text{-}3\text{-}CO_2C_2H_5\text{-}C_6H_3$ | | 3 |
| 169 | " | $4\text{-}CH_3\text{-}3\text{-}CO_2C_2H_5\text{-}C_6H_3$ | | 3 |
| 170 | " | $4\text{-}OC_2H_5\text{-}3\text{-}CO_2C_2H_5\text{-}C_6H_3$ | | 3 |
| 171 | " | $4\text{-}OCH_3\text{-}3\text{-}CO_2C_2H_5\text{-}C_6H_3$ | | 3 |
| 172 | " | $2\text{-}F\text{-}4\text{-}Cl\text{-}5\text{-}CO_2C_2H_5\text{-}C_6H_2$ | | 3 |
| 173 | $\overset{\overset{O}{\parallel}}{P}[OCH(CH_3)_2]_2$ | $4\text{-}F\text{-}3\text{-}CO_2C_2H_5\text{-}C_6H_3$ | | 3 |
| 174 | " | $4\text{-}Cl\text{-}3\text{-}CO_2C_2H_5\text{-}C_6H_3$ | | 3 |
| 175 | " | $4\text{-}Br\text{-}3\text{-}CO_2C_2H_5\text{-}C_6H_3$ | | 3 |
| 176 | $\overset{\overset{O}{\parallel}}{P}(OC_4H_9)_2$ | $4\text{-}F\text{-}3\text{-}CO_2C_2H_5\text{-}C_6H_3$ | | 3 |
| 177 | " | $4\text{-}Cl\text{-}3\text{-}CO_2C_2H_5\text{-}C_6H_3$ | | 3 |
| 178 | " | $4\text{-}Br\text{-}3\text{-}CO_2C_2H_5\text{-}C_6H_3$ | | 3 |
| 179 | $\overset{\overset{O}{\parallel}}{P}(OC_2H_5)_2$ | $4\text{-}Br\text{-}3\text{-}[CO_2CH(CH_3)CO_2C_2H_5]\text{-}C_6H_3$ | | 3 |
| 180 | " | $4\text{-}Cl\text{-}3\text{-}[CO_2CH(CH_3)CO_2C_2H_5]\text{-}C_6H_3$ | | 3 |
| 181 | " | $4\text{-}F\text{-}3\text{-}[CO_2CH(CH_3)CO_2C_2H_5]\text{-}C_6H_3$ | | 3 |
| 182 | " | $2,4\text{-}F_2\text{-}5\text{-}[CO_2CH(CH_3)CO_2C_2H_5]\text{-}C_6H_2$ | | 3 |
| 183 | " | $4\text{-}Br\text{-}3\text{-}CO_2CH_2CF_3\text{-}C_6H_3$ | | 3 |
| 184 | $\overset{\overset{O}{\parallel}}{P}(OCH_3)_2$ | $4\text{-}Cl\text{-}3\text{-}CO_2CH_2CF_3\text{-}C_6H_3$ | | 3 |
| 185 | " | $4\text{-}F\text{-}3\text{-}CO_2CH_2CF_3\text{-}C_6H_3$ | | 3 |
| 186 | $\underset{CH_3}{\overset{\overset{O}{\parallel}}{P}}\text{-}OC_2H_5$ | $4\text{-}F\text{-}3\text{-}CO_2CH_2CF_3\text{-}C_6H_3$ | | 133 |

| Bsp. | A | $R^2$ | Fp[°C] | hergestellt analog Bsp. |
|---|---|---|---|---|
| 187 | $\overset{O}{\underset{CH_3}{P}}-OC_2H_5$ | 4-F-3-$CO_2C_2H_5$-$C_6H_3$ | | 133 |
| 188 | " | 4-Br-3-$CO_2C_2H_5$-$C_6H_3$ | | 133 |
| 189 | " | 2,4-$F_2$-3-$CO_2C_2H_5$-$C_6H_3$ | | 133 |
| 190 | " | 2-F-4-Cl-5-$CO_2C_2H_5$-$C_6H_2$ | | 133 |
| 191 | " | 4-Cl-3-[$CO_2CH(CH_3)$-$CO_2C_2H_5$]-$C_6H_3$ | | 133 |
| 192 | " | 4-Br-3-[$CO_2CH(CH_3)CO_2C_2H_5$]-$C_6H_3$ | | 133 |
| 193 | " | 4-F-3-[$CO_2CH(CH_3)CO_2C_2H_5$]-$C_6H_3$ | | 133 |
| 194 | " | 2,4-$F_2$-5-[$CO_2CH(CH_3)CO_2C_2H_5$]-$C_6H_2$ | | 133 |
| 195 | $\overset{O}{P}(C_6H_5)_2$ | 4-Cl-3-$CO_2C_2H_5$-$C_6H_3$ | | 136 |
| 196 | " | 4-F-3-$CO_2C_2H_5$-$C_6H_3$ | | 136 |
| 197 | " | 4-Br-3-$CO_2C_2H_5$-$C_6H_3$ | | 136 |
| 198 | " | 2,4-$F_2$-3-$CO_2C_2H_5$-$C_6H_2$ | | 136 |
| 199 | " | 4-Br-3-[$CO_2CH(CH_3)$-$CO_2C_2H_5$]-$C_6H_3$ | | 136 |
| 200 | $\overset{O}{\underset{CH_3}{P}}-C_6H_5$ | 4-Cl-3-$CO_2C_2H_5$-$C_6H_3$ | | 136 |
| 201 | " | 4-Br-3-$CO_2C_2H_5$-$C_6H_3$ | | 136 |
| 202 | " | 1,4-$F_2$-3-$CO_2C_2H_5$-$C_6H_2$ | | 136 |
| 203 | " | 4-Cl-3-[$CO_2CH(CH_3)$-$CO_2C_2H_5$]-$C_6H_3$ | | 136 |
| 204 | $\overset{O}{P}(OC_2H_5)_2$ | 4-Cl-2F-5-($OCH_2C\equiv C$)-$C_6H_2$ | 124-127 | 3 |
| 205 | " | 4-Br-2F-5-$OCH_3$-$C_6H_2$ | 146 | 3 |

## C. Biologische Beispiele

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist. Dabei bedeutet:

0 = ohne Wirkung bzw. Schaden
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

### 1. Unkrautwirkung im Vorauflauf

Samen bzw. Rhizomstücke von mono- und dikotylen Unkrautpflanzen wurden in Plastiktöpfen (Ø = 9 cm) in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 l/ha in unterschiedlichen Dosierungen auf die Oberfläche der Abdeckerde appliziert.
Nach der Behandlung wurden die Töpfe im Gewächshaus aufgestellt und unter guten Wachstumsbedingungen für die Unkrautpflanzen gehalten (Temperatur 23 plus/minus 1 °C, relative Luftfeuchte 60 - 80 %).

Die optische Bonitur der Pflanzen- bzw. Auflaufschäden erfolgte nach dem Auflaufen der Versuchspflanzen nach einer Versuchszeit von 3 - 4 Wochen im Vergleich zu unbehandelten Kontrollen.
Die Boniturwerte in Tabelle 2 zeigen, daß die erfindungsgemäßen Verbindungen eine gute herbizide Vorauflaufwirksamkeit gegen ein breites Spektrum von Ungräsern und Unkräutern besitzen.

Tabelle 2

Vorauflaufwirkung der erfindungsgemäßen Verbindungen

| Bsp. | Dosis kg a.i./ha | herbizide Wirkung SIA | CRS | LOM | ECG |
|---|---|---|---|---|---|
| 5 | 2,5 | 5 | 5 | 5 | 5 |
| 49 | 2,5 | 5 | 5 | 5 | 5 |
| 50 | 2,5 | 3 | 4 | 5 | 5 |
| 3 | 2,5 | 5 | 5 | 5 | 5 |
| 6 | 2,5 | 4 | 5 | 5 | 4 |
| 2 | 2,5 | 3 | 4 | 5 | 5 |
| 1 | 2,5 | 5 | 5 | 5 | 5 |
| 8 | 2,5 | 5 | 5 | 5 | 5 |
| 53 | 2,5 | 4 | 5 | 5 | 5 |
| 4 | 2,5 | 5 | 5 | 5 | 5 |
| 20 | 2,5 | 4 | 5 | 4 | 4 |
| 67 | 2,5 | 2 | 5 | 5 | 4 |
| 85 | 2,5 | 3 | 5 | 5 | 5 |
| 87 | 2,5 | 4 | 5 | 4 | 5 |
| 108 | 2,5 | 5 | 5 | 5 | 4 |
| 109 | 2,5 | 5 | 5 | 5 | 5 |
| 114 | 2,5 | 4 | 5 | 5 | 5 |
| 125 | 2,5 | 3 | 5 | 4 | 3 |
| 129 | 2,5 | 5 | 5 | 5 | 5 |
| 131 | 2,5 | 5 | 5 | 3 | 5 |
| 132 | 2,5 | 2 | 3 | 5 | 3 |
| 133 | 2,5 | 5 | 5 | 5 | 5 |
| 134 | 2,5 | 5 | 5 | 5 | 5 |
| 136 | 2,5 | 5 | 5 | 5 | 5 |
| 139 | 2,5 | 5 | 5 | 5 | 5 |
| 140 | 2,5 | 1 | 1 | 5 | 1 |
| 142 | 2,5 | 4 | 5 | 4 | 5 |
| 143 | 2,5 | 2 | 3 | 5 | 5 |
| 144 | 2,5 | 5 | 5 | 5 | 5 |
| 204 | 2,5 | 5 | 5 | 5 | 5 |
| 205 | 2,5 | 5 | 5 | 5 | 5 |

Abkürzungen:

SIA = Sinapis arvensis

CRS = Chrysanthemum segetum

LOM = Lolium multiflorum

ECG = Echinochloa crus-galli

a. i.= Aktivsubstanz

## 2. Unkrautwirkung im Nachauflauf

Samen bzw. Rhizomstücke von mono- und dikotylen Unkräutern wurden in Plastiktöpfen (Ø = 9 cm) in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 l/ha auf die grünen Pflanzenteile gesprüht und nach ca. 3 - 4 Wochen Standzeit der Versuchspflanzen im Gewächshaus unter optimalen Wachstumsbedingungen (Temperatur 23 plus/minus 1 °C, relative Luftfeuchte 60 - 80 %) die Wirkung der Präparate optisch im Vergleich zu unbehandelten Kontrollen bonitiert.

Die erfindungsgemäßen Verbindungen weisen auch im Nachauflauf eine gute herbizide Wirksamkeit gegen ein breites Spektrum wirtschaftlich wichtiger Ungräser und Unkräuter auf (Tabelle 3).

0215425

Tabelle 3 Nachlaufwirkung der erfindungsgemäßen Verbindungen

| Bsp. | Dosis kg a.i./ha | herbizide Wirkung SIA | CRS | LOM | ECG |
|---|---|---|---|---|---|
| 5 | 2,5 | 4 | 3 | 4 | 2 |
| 49 | 2,5 | 5 | 4 | 5 | 5 |
| 3 | 2,5 | 5 | 5 | 5 | 4 |
| 1 | 2,5 | 5 | 4 | 3 | 2 |
| 8 | 2,5 | 4 | 4 | 3 | 3 |
| 4 | 2,5 | 5 | 5 | 5 | 5 |
| 67 | 2,5 | 3 | 4 | 1 | 2 |
| 87 | 2,5 | 3 | 4 | 3 | 5 |
| 109 | 2,5 | 5 | 3 | 4 | 2 |
| 114 | 2,5 | 4 | 3 | 4 | 2 |
| 125 | 2,5 | 5 | 5 | 1 | 2 |
| 129 | 2,5 | 5 | 5 | 5 | 5 |
| 131 | 2,5 | 5 | 2 | 5 | 4 |
| 132 | 2,5 | 5 | 2 | 5 | 2 |
| 133 | 2,5 | 5 | 5 | 5 | 5 |
| 134 | 2,5 | 5 | 5 | 5 | 5 |
| 136 | 2,5 | 5 | 3 | 5 | 5 |
| 139 | 2,5 | 2 | 2 | 5 | 4 |
| 142 | 2,5 | 5 | 1 | 4 | 1 |
| 143 | 2,5 | 5 | 1 | 5 | 2 |
| 144 | 2,5 | 4 | 1 | 5 | 2 |
| 204 | 2,5 | 5 | 5 | 5 | 5 |
| 205 | 2,5 | 5 | 5 | 5 | 5 |

Abkürzungen: s. Tab. 2

## 3. Kulturpflanzenverträglichkeit

In weiteren Versuchen im Gewächshaus wurden Samen einer größeren Anzahl von Kulturpflanzen in Töpfen in sandigem Lehmboden ausgelegt und mit Erde abgedeckt.
Ein Teil der Töpfe wurde sofort wie unter 1. beschrieben behandelt; die übrigen wurden im Gewächshaus aufgestellt, bis die Pflanzen zwei bis drei echte Blät-

0215425

ter entwickelt hatten und dann mit den erfindungsgemäßen Substanzen in unterschiedlichen Dosierungen, wie unter 2. beschrieben, besprüht.
Vier bis fünf Wochen nach der Applikation und Standzeit im Gewächshaus wurde mittels optischer Bonitur festgestellt, daß die erfindunsgemäßen Verbindungen zweikeimblättrige Kulturen wie z. B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z. B. Gerste, Sorghum, Mais, Weizen oder Reis. Die Verbindungen der Formel I weisen somit eine hohe Selektivität bei Anwendung zur Bekämpfung von unerwünschtem Pflanzenwuchs in landwirtschaftlich wichtigen Kulturen auf.

0215425

Patentansprüche:

1. Verbindungen der Formel I

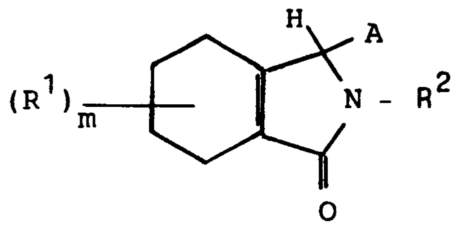

(I)

worin

$R^1$ = $(C_1-C_4)$Alkyl;

$R^2$ = Phenyl, Naphthyl, Pyridyl, Chinolinyl oder Isochinolinyl bedeutet, wobei diese Reste ein- bis vierfach, vorzugsweise ein- bis dreifach, durch gleiche oder verschiedene Reste der Gruppe Halogen, Hydroxy, $(C_1-C_4)$Alkyl, $(C_1-C_4)$Alkoxy, $(C_1-C_4)$Alkoxy-$(C_1-C_4)$alkoxy, $(C_3-C_6)$Cycloalkoxy, $(C_3-C_6)$Alkenyloxy, $(C_3-C_6)$Alkinyloxy, Halogen-$(C_1-C_4)$alkyl, Halogen$(C_1-C_4)$alkoxy, Halogen $(C_3-C_6)$-alkenyloxy, Halogen $(C_3-C_6)$alkinyloxy, $(C_1-C_4)$-Alkylthio, $(C_1-C_4)$Alkylsulfinyl, $(C_1-C_4)$-Alkylsulfonyl, wobei bei den drei letztgenannten Resten der Alkylrest durch $(C_1-C_4)$-Alkoxycarbonyl substituiert sein kann, $NO_2$, CN, Halogen$(C_1-C_4)$alkyl, Cyano$(C_1-C_4)$alkyl, Phenoxy, Phenoxy$(C_1-C_4)$-alkyl, Phenyl$(C_1-C_4)$alkoxy, Phenylthio, Phenylsulfinyl, Phenylsulfonyl, wobei die sechs letztgenannten Reste im Phenylring bis zu dreifach durch Halogen, $(C_1-C_4)$Alkyl, $(C_1-C_4)$-Alkoxy oder $(C_1-C_4)$Alkoxycarbonyl substituiert sein können, oder durch einen Rest der Formeln

$-\underset{\underset{O}{\|}}{C}-X-R^3$, $-O-\overset{\overset{Z}{\|}}{P}(YR^4)_2$ oder $-Z-(\overset{\overset{R^5}{|}}{C}H)_n-\overset{\overset{O}{\|}}{C}-X-R^3$

substituiert sein können,

X = O, S oder $NR^6$,

Y,Z = unabhängig voneinander O oder S,

$R^3$ = Wasserstoff, $(C_1-C_{12})$Alkyl, das bis zu 6-fach durch Halogen und/oder bis zu zweifach durch $(C_1-C_4)$-Alkoxy, $(C_1-C_4)$Alkoxy$(C_1-C_4)$alkoxy, $(C_1-C_4$-Alkoxy)-carbonyl, $(C_1-C_4)$Alkylthio, $(C_1-C_4)$Alkylsulfinyl, $(C_1-C_4)$Alkylsulfonyl, $(C_1-C_4)$Alkylamino, Di$(C_1-C_4)$alkylamino, Furyl, Tetrahydrofuryl, Benzofuryl, Phenyl, Phenoxy, Benzyloxy, wobei die Phenylringe bis zu dreifach durch Halogen, $(C_1-C_4)$Alkyl oder $(C_1-C_4)$Alkoxy substituiert sein können, substituiert sein kann,
$(C_3-C_6)$Cycloalkyl, $(C_3-C_6)$Alkenyl, Cyclohexenyl, $(C_3-C_6)$Alkinyl, Phenyl, das bis zu dreifach substituiert sein kann durch Halogen, $(C_1-C_4)$Alkyl, $(C_1-C_4)$Alkoxy oder $(C_1-C_4$Alkoxy)-carbonyl;
einen Rest der Formel

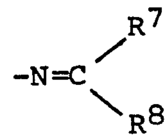

oder ein in der Landwirtschaft einsetzbares Kation;

$R^4$ = $(C_1-C_4)$Alkyl,

$R^5$ = Wasserstoff, $(C_1-C_4)$Alkyl,

$R^6$ = Wasserstoff, $(C_1-C_4)$Alkyl, das zusammen mit $R^3$ und dem diese Reste verbindenden Stickstoffatom einen fünf- bis siebengliedrigen Heterocyclus bilden kann, der als Ringglieder ein oder zwei Reste der Gruppe -O-, -S-, $NR^5$ enthält und bis zu dreifach durch $(C_1-C_4)$Alkyl substituiert sein kann,

$R^7, R^8$ = unabhängig voneinander $(C_1-C_4)$Alkyl, $(C_3-C_5)$Cycloalkyl oder gemeinsam eine $(C_2-C_7)$Alkylenkette;

A = einen Rest der Formeln

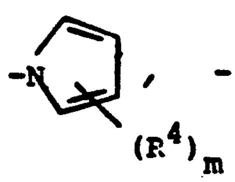 , 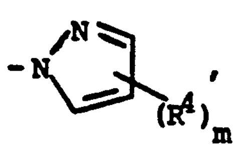 , 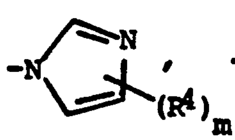 , 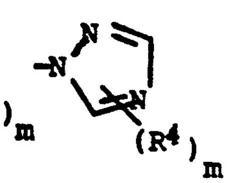

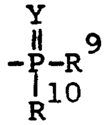 oder -NCS,

$R^9$, $R^{10}$ unabhängig voneinander $(C_1-C_4)$Alkyl, Phenyl oder Benzyl, die beide bis zu zweifach durch Halogen, $(C_1-C_4)$Alkyl, $CF_3$ oder $(C_1-C_4)$Alkoxy substituiert sein können; $(C_1-C_4)$Alkoxy; $(C_1-C_4)$-Alkylthio; OH, SH, OM oder SM, wobei M ein in der Landwirtschaft einsetzbares Kation bedeutet, oder einen Rest der Formel $-NR^{11}R^{12}$,

$R^{11}$, $R^{12}$ unabhängig voneinander Wasserstoff; $(C_1-C_6)$-Alkyl; Phenyl oder Benzyl, die beide bis zu zweifach durch Halogen, $(C_1-C_4)$Alkyl, $CF_3$ oder $NO_2$ substituiert sein können, oder $R^{11}$ und $R^{12}$ zusammen mit dem benachbarten N-Atom einen fünf- bis sechsgliedrigen Heterocyclus, der als Ringglieder ein oder zwei Reste der Gruppe -O-, -S- oder $-NR^5-$ enthalten kann und der bis zu dreifach durch $(C_1-C_4)$-Alkyl substituiert sein kann;

m = 0, 1 oder 2 und

n = 1, 2 oder 3

bedeuten.

2. Verbindungen der Formel I, worin

$R^2$ = Phenyl, Pyridyl, die beide 1- bis 3-fach durch $(C_1-C_4)$Alkyl, das bis zu dreifach durch Halogen substituiert sein kann, durch F, Cl, Br, $(C_1-C_4)$-Alkoxy, das bis zu neunfach durch Fluor und/oder Chlor substituiert sein kann, durch $(C_1-C_4)$Alkylthio, $-CO-X-R^3$ oder $-Z-(\underset{R^5}{\underset{|}{C}}H)_n-CO-X-R^3$ substituiert sind,

X, Y, Z = O oder S,

$R^3$ = $(C_1-C_6)$Alkyl, $(C_1-C_4)$Alkoxycarbonyl-$(C_1-C_4)$alkyl oder $(C_1-C_4)$Alkoxy-$(C_1-C_4)$alkyl,

A = einen Rest der Formeln

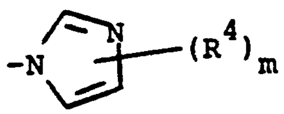 , 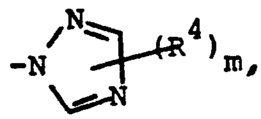 , 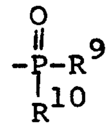 oder -NCS,

$R^9$, $R^{10}$ = unabhängig voneinander $(C_1-C_4)$Alkyl; Phenyl oder Benzyl, die beide bis zu zweifach durch Halogen, $(C_1-C_4)$Alkyl, $CF_3$ oder $(C_1-C_4)$Alkoxy substituiert sein können; $(C_1-C_4)$Alkoxy; $(C_1-C_4)$-Alkylthio; OH, SH, OM oder SM, wobei M ein in der Landwirtschaft einsetzbares Kation bedeutet, oder einen Rest der Formel $-NR^{11}R^{12}$ und

$R^{11}$, $R^{12}$ = unabhängig voneinander Wasserstoff; $(C_1-C_6)$-Alkyl; Phenyl oder Benzyl, die beide bis zu dreifach durch Halogen, $(C_1-C_4)$Alkyl, $CF_3$ oder $NO_2$ substituiert sein können oder $R^{11}$ und $R^{12}$ zusammen mit dem benachbarten N-Atom einen fünf- bis sechsgliedrigen Heterocyclus, der als Ringglieder ein oder zwei Reste der Gruppe -O-, -S- oder $-NR^5-$ enthalten kann und der bis zu dreifach durch $(C_1-C_4)$-Alkyl substituiert sein kann;

n = 1,
m = 0 oder 1 bedeuten.

3. Verbindungen gemäß Formel I von Anspruch 1, dadurch gekennzeichnet, daß

$R^2$ = Phenyl, das 1- bis 3-fach durch $(C_1-C_4)$Alkyl, das bis zu dreifach durch Halogen substituiert sein kann, F, Cl, Br, $(C_1-C_4)$Alkoxy, das bis zu dreifach durch Fluor und/oder Chlor substituiert sein kann, $(C_1-C_4)$Alkylthio, $-CO-X-R^3$ oder

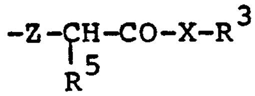

0215425

substituiert ist, wobei X, Z = O oder S, $R^3$ = $(C_1-C_6)$Alkyl, $(C_1-C_4)$-Alkoxycarbonyl-$(C_1-C_4)$alkyl oder $(C_1-C_4)$Alkoxy-$(C_1-C_4)$alkyl; $R^5$ = H oder $(C_1-C_2)$Alkyl, bedeuten

A = einen Rest der Formel $-P(O)R^9R^{10}$, wobei

$R^9$, $R^{10}$ = unabhängig voneinander $(C_1-C_4)$Alkyl, Phenyl, OH oder OM bedeuten, und m für O steht,

bedeuten.

4. Verfahren zur Herstellung der Verbindungen der Formel I von Ansprüchen 1, 2 oder 3, dadurch gekennzeichnet, daß man eine Verbindung der Formel II

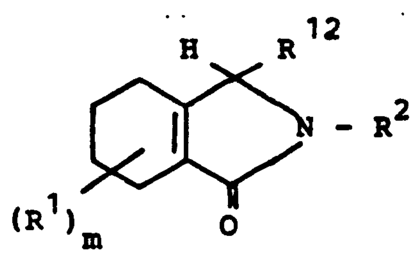

(II),

worin $R^{12}$ eine nucleofuge Gruppe wie Halogen, Tosyl oder Mesyl bedeutet, mit einer Verbindung der Formel H-A', M-SCN oder

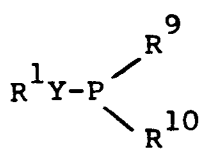

, wobei A' die Bedeutung von A außer

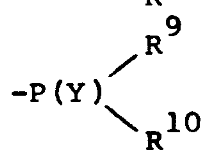

und SCN besitzt und M ein Metallkation bedeutet,

umsetzt, und die erhaltenen Verbindungen gewünschtenfalls derivatisiert.

5. Verwendung der Verbindungen der Formel I von Ansprüchen 1, 2 oder 3 als Herbizide.

6. Herbizide Mittel, dadurch gekennzeichnet, daß sie eine Verbindung der Formel I von Anspruch 1, 2 oder 3 enthalten.

7. Verfahren zur Bekämpfung von unerwünschten Pflanzen in Nutzpflanzenkulturen, dadurch gekennzeichnet, daß man auf die Schadpflanzen oder die Anbaufläche eine wirksame Menge einer Verbindung der Formel I von Ansprüchen 1, 2 oder 3 aufbringt.

Patentansprüche: (Vertragsstaat AT)

1. Verfahren zur Herstellung der Verbindungen der Formel I

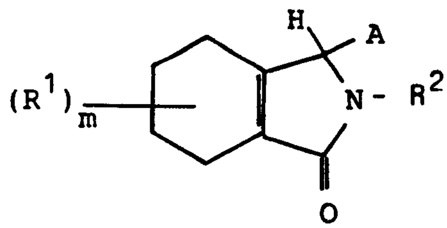

(I)

worin

$R^1$ = $(C_1-C_4)$Alkyl;

$R^2$ = Phenyl, Naphthyl, Pyridyl, Chinolinyl oder Isochinolinyl bedeutet, wobei diese Reste ein- bis vierfach, vorzugsweise ein- bis dreifach, durch gleiche oder verschiedene Reste der Gruppe Halogen, Hydroxy, $(C_1-C_4)$Alkyl, $(C_1-C_4)$Alkoxy, $(C_1-C_4)$-Alkoxy-$(C_1-C_4)$alkoxy, $(C_3-C_6)$Cycloalkoxy, $(C_3-C_6)$Alkenyloxy, $(C_3-C_6)$Alkinyloxy, Halogen-$(C_1-C_4)$alkyl, Halogen$(C_1-C_4)$alkoxy, Halogen $(C_3-C_6)$-alkenyloxy, Halogen $(C_3-C_6)$alkinyloxy, $(C_1-C_4)$-Alkylthio, $(C_1-C_4)$Alkylsulfinyl, $(C_1-C_4)$-Alkylsulfonyl, wobei bei den drei letztgenannten Resten der Alkylrest durch $(C_1-C_4)$-Alkoxycarbonyl substituiert sein kann, $NO_2$, CN, Halogen$(C_1-C_4)$alkyl, Cyano$(C_1-C_4)$alkyl, Phenoxy, Phenoxy$(C_1-C_4)$-alkyl, Phenyl$(C_1-C_4)$alkoxy, Phenylthio, Phenylsulfinyl, Phenylsulfonyl, wobei die sechs letztgenannten Reste im Phenylring bis zu dreifach durch Halogen, $(C_1-C_4)$Alkyl, $(C_1-C_4)$Alkoxy oder $(C_1-C_4)$Alkoxycarbonyl substituiert sein können, oder durch einen Rest der Formeln

$-\underset{O}{\underset{\|}{C}}-X-R^3$, $-O-\overset{Z}{\overset{\|}{P}}(YR^4)_2$ oder $-Z-(\overset{R^5}{\overset{|}{C}}H)_n-\overset{O}{\overset{\|}{C}}-X-R^3$

substituiert sein können,

X = O, S oder $NR^6$,

Y,Z = unabhängig voneinander O oder S,

$R^3$ = Wasserstoff, $(C_1-C_{12})$Alkyl, das bis zu 6-fach durch Halogen und/oder bis zu zweifach durch $(C_1-C_4)$-Alkoxy, $(C_1-C_4)$Alkoxy$(C_1-C_4)$alkoxy, $(C_1-C_4$-Alkoxy)-carbonyl, $(C_1-C_4)$Alkylthio, $(C_1-C_4)$Alkylsulfinyl, $(C_1-C_4)$Alkylsulfonyl, $(C_1-C_4)$Alkylamino, Di$(C_1-C_4)$-alkylamino, Furyl, Tetrahydrofuryl, Benzofuryl, Phenyl, Phenoxy, Benzyloxy, wobei die Phenylringe bis zu dreifach durch Halogen, $(C_1-C_4)$Alkyl oder $(C_1-C_4)$Alkoxy substituiert sein können, substituiert sein kann,
$(C_3-C_6)$Cycloalkyl, $(C_3-C_6)$Alkenyl, Cyclohexenyl, $(C_3-C_6)$Alkinyl, Phenyl, das bis zu dreifach substituiert sein kann durch Halogen, $(C_1-C_4)$Alkyl, $(C_1-C_4)$Alkoxy oder $(C_1-C_4$Alkoxy)-carbonyl;
einen Rest der Formel 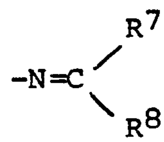

oder ein in der Landwirtschaft einsetzbares Kation;

$R^4$ = $(C_1-C_4)$Alkyl,

$R^5$ = Wasserstoff, $(C_1-C_4)$Alkyl,

$R^6$ = Wasserstoff, $(C_1-C_4)$Alkyl, das zusammen mit $R^3$ und dem diese Reste verbindenden Stickstoffatom einen fünf- bis siebengliedrigen Heterocyclus bilden kann, der als Ringglieder ein oder zwei Reste der Gruppe -O-, -S-, $NR^5$ enthält und bis zu dreifach durch $(C_1-C_4)$Alkyl substituiert sein kann,

$R^7$,$R^8$ =unabhängig voneinander $(C_1-C_4)$Alkyl, $(C_3-C_6)$Cycloalkyl oder gemeinsam eine $(C_2-C_7)$Alkylenkette;

A = einen Rest der Formeln

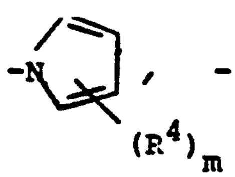 , 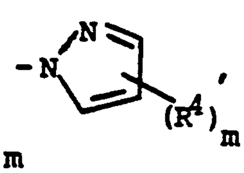 , 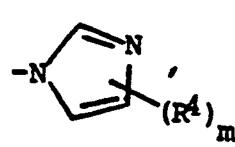 , 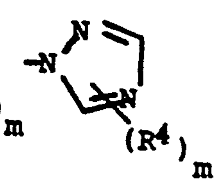

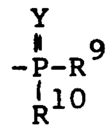 oder -NCS,

$R^9$, $R^{10}$ unabhängig voneinander $(C_1-C_4)$Alkyl, Phenyl oder Benzyl, die beide bis zu zweifach durch Halogen, $(C_1-C_4)$Alkyl, $CF_3$ oder $(C_1-C_4)$Alkoxy substituiert sein können; $(C_1-C_4)$Alkoxy; $(C_1-C_4)$-Alkylthio; OH, SH, OM oder SM, wobei M ein in der Landwirtschaft einsetzbares Kation bedeutet, oder einen Rest der Formel $-NR^{11}R^{12}$,

$R^{11}$, $R^{12}$ unabhängig voneinander Wasserstoff; $(C_1-C_6)$Alkyl; Phenyl oder Benzyl, die beide bis zu zweifach durch Halogen, $(C_1-C_4)$Alkyl, $CF_3$ oder $NO_2$ substituiert sein können, oder $R^{11}$ und $R^{12}$ zusammen mit dem benachbarten N-Atom einen fünf- bis sechsgliedrigen Heterocyclus, der als Ringglieder ein oder zwei Reste der Gruppe -O-, -S- oder $-NR^5-$ enthalten kann und der bis zu dreifach durch $(C_1-C_4)$-Alkyl substituiert sein kann;

m = 0, 1 oder 2 und

n = 1, 2 oder 3

bedeuten, dadurch gekennzeichnet, daß man eine Verbindung der Formel II

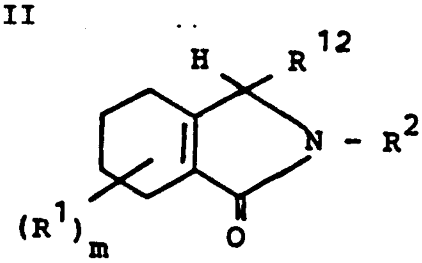

(II),

worin $R^{12}$ eine nucleofuge Gruppe wie Halogen, Tosyl oder Mesyl bedeutet, mit einer Verbindung der Formel H-A', M-SCN oder

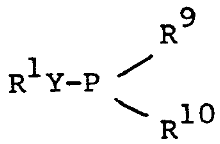 , wobei A' die Bedeutung von A außer

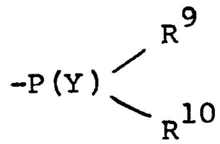 und SCN- besitzt und M ein Metallkation, bevorzugt ein Blei(II), Kalium- oder Na-Ion, bedeutet, umsetzt, und die erhaltenen Verbindungen gewünschtenfalls derivatisiert.

2. Verwendung der Verbindungen der Formel I von Anspruch 1 als Herbizid.

3. Herbizide Mittel, dadurch gekennzeichnet, daß sie eine Verbindung der Formel I von Anspruch 1 enthalten.

4. Herbizide Mittel, dadurch gekennzeichnet, daß sie eine Verbindung der Formel I,
worin

$R^2$ = Phenyl, Pyridyl, die beide 1- bis 3-fach durch $(C_1-C_4)$Alkyl, das bis zu dreifach durch Halogen substituiert sein kann, durch F, Cl, Br, $(C_1-C_4)$-Alkoxy, das bis zu neunfach durch Fluor und/oder Chlor substituiert sein kann, durch $(C_1-C_4)$Alkylthio, $-CO-X-R^3$ oder $-Z-(\underset{R^5}{CH})_n-CO-X-R^3$ substituiert sind,

X, Y, Z = O oder S,

$R^3$ = $(C_1-C_6)$Alkyl, $(C_1-C_4)$Alkoxycarbonyl-$(C_1-C_4)$alkyl oder $(C_1-C_4)$Alkoxy-$(C_1-C_4)$alkyl,

A = einen Rest der Formeln

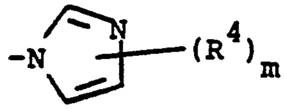 , 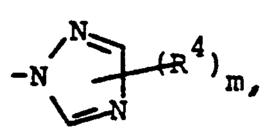 , 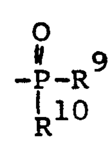 oder -NCS,

$R^9$, $R^{10}$ = unabhängig voneinander $(C_1-C_4)$Alkyl; Phenyl oder Benzyl, die beide bis zu zweifach durch Halogen, $(C_1-C_4)$Alkyl, $CF_3$ oder $(C_1-C_4)$Alkoxy substituiert sein können; $(C_1-C_4)$Alkoxy; $(C_1-C_4)$-Alkylthio; OH, SH, OM oder SM, wobei M ein in der Landwirtschaft einsetzbares Kation bedeutet, oder einen Rest der Formel $-NR^{11}R^{12}$ und

$R^{11}$, $R^{12}$ = unabhängig voneinander Wasserstoff; $(C_1-C_6)$-Alkyl; Phenyl oder Benzyl, die beide bis zu dreifach durch Halogen, $(C_1-C_4)$Alkyl, $CF_3$ oder $NO_2$ substituiert sein können oder $R^{11}$ und $R^{12}$ zusammen mit dem benachbarten N-Atom einen fünf- bis sechsgliedrigen Heterocyclus, der als Ringglieder ein oder zwei Reste der Gruppe -O-, -S- oder $-NR^5-$ enthalten kann und der bis zu dreifach durch $(C_1-C_4)$-Alkyl substituiert sein kann;

n = 1,
m = 0 oder 1 bedeuten,
enthalten.

5. Herbizide Mittel, dadurch gekennzeichnet, daß sie eine Verbindung der Formel I, worin

$R^2$ = Phenyl, das 1- bis 3-fach durch $(C_1-C_4)$Alkyl, das bis zu dreifach durch Halogen substituiert sein kann, F, Cl, Br, $(C_1-C_4)$Alkoxy, das bis zu dreifach durch Fluor und/oder Chlor substituiert sein kann, $(C_1-C_4)$Alkylthio, $-CO-X-R^3$ oder

$$-Z-\underset{\displaystyle R^5}{\underset{|}{CH}}-CO-X-R^3$$

substituiert ist,
wobei X, Z = O oder S, $R^3$ = $(C_1-C_6)$Alkyl, $(C_1-C_4)$-

Alkoxycarbonyl-$(C_1-C_4)$alkyl oder $(C_1-C_4)$Alkoxy-$(C_1-C_4)$alkyl; $R^5$ = H oder $(C_1-C_2)$Alkyl, bedeuten

A = einen Rest der Formel $-P(O)R^9R^{10}$, wobei

$R^9$, $R^{10}$ = unabhängig voneinander $(C_1-C_4)$Alkyl, Phenyl, OH oder OM bedeuten, und m für O steht,

bedeuten,

enthalten.

6. Verfahren zur Bekämpfung von unerwünschten Pflanzen in Nutzpflanzenkulturen, dadurch gekennzeichnet, daß man auf diese oder die Anbaufläche eine wirksame Menge einer Verbindung der Formel I von Anspruch 1, 4 oder 5 aufbringt.